(19) **Europäisches Patentamt**
**European Patent Office**
**Office européen des brevets**

(11) **EP 1 479 342 B1**

(12) **EUROPEAN PATENT SPECIFICATION**

(45) Date of publication and mention
of the grant of the patent:
**30.09.2009 Bulletin 2009/40**

(51) Int Cl.:
*A61B 5/18* (2006.01)     *B60K 28/06* (2006.01)
*G06F 17/00* (2006.01)     *G08B 21/06* (2006.01)

(21) Application number: **04012089.1**

(22) Date of filing: **21.05.2004**

(54) **Mental state assessment apparatus and mental state assessment method**

Vorrichtung und Verfahren zur Bestimmung des geistigen Zustands

Appareil et procédé pour déterminer la condition mentale

(84) Designated Contracting States:
**DE FR GB**

(30) Priority: **21.05.2003   JP 2003142935**

(43) Date of publication of application:
**24.11.2004   Bulletin 2004/48**

(73) Proprietor: **Pioneer Corporation
Tokyo-to (JP)**

(72) Inventors:
• **Yanagidaira, Masatoshi
Tsurugashima-shi
Saitama-ken (JP)**

• **Yasushi, Misuo
Tsurugashima-shi
Saitama-ken (JP)**

(74) Representative: **Reinhard - Skuhra - Weise &
Partner GbR
Patent- und Rechtsanwälte
P.O. Box 440151
80750 München (DE)**

(56) References cited:
**DE-A- 4 400 207          JP-A- 11 151 231**

**Description**

BACKGROUND OF THE INVENTION

Field of the Invention

The present invention relates to a mental state assessment apparatus and mental state assessment method which assess mental state of a person or animal based on his heartbeats.

Related Art

Body condition detector have been proposed which detects wakeful state of a vehicle driver based on biomedical information including an average heart rate, cardiac cycle, respiration rate, average respiratory cycle, etc. of the vehicle driver (See, for example, JP-H07-059757A or DE 44 00 207A). Such a body condition detector compares an average cardiac cycle measured from the driver with a predetermined reference value and judges wakeful state of the driver based on a comparison result.

However, since man's mental state changes at any time even in a normal state depending on external environment, time of day, etc., methods such as the one described above cannot correctly assess a transition from normal state to sleepy state.

SUMMARY OF THE INVENTION

Therefore, an object of the present invention is to provide a new mental state assessment apparatus and mental state assessment method which each can solve the above mentioned problems. Another object of the present invention is to provide a mental state assessment apparatus and mental state assessment method which each can correctly assess mental state of a subject irrespective of external environment or time of day.

The present invention provides a mental state assessment apparatus which assesses mental state of a subj ect, and comprises the features of claim 1.

The present invention also provides a mental state assessment method for assessing mental state of a subject, which comprises the features of claim 6.

BRIEF DESCRIPTION OF THE DRAWINGS

FIG. 1 is a diagram showing a configuration of a mental state assessment apparatus according to an embodiment of the present invention;
FIG. 2 is a diagram showing a flow of a mental state assessment process performed by a mental state assessment section 15 shown in FIG. 1;
FIG. 3 is a diagram showing a flow of a subroutine for detecting a state transition from normal state;

FIG. 4 is a diagram showing a flow of a subroutine for detecting a state transition from tense state;
FIG. 5 is a diagram showing a flow of a subroutine for detecting a state transition from sleepy state;
FIG. 6 is a diagram illustrating operation of the mental state assessment apparatus shown in FIG. 1;
FIG. 7 is a diagram showing a heart rate downward trend detection subroutine;
FIGS. 8A and 8B are diagrams showing examples of drops in heart rate during a transition to sleepy state, heart rate data CN1 to CN6 which depicts a falling heart rate, and falls a1 to a3 and variations b1 and b2; and
FIG. 9 is a diagram showing an example of a sleepiness assessment subroutine executed instead of Steps S77 to S80 shown in FIG. 3.

DETAILED DESCRIPTION OF THE PREFERRED EMBODIMENTS

An embodiment of the present invention will be described in detail below with reference to the drawings annexed hereto.

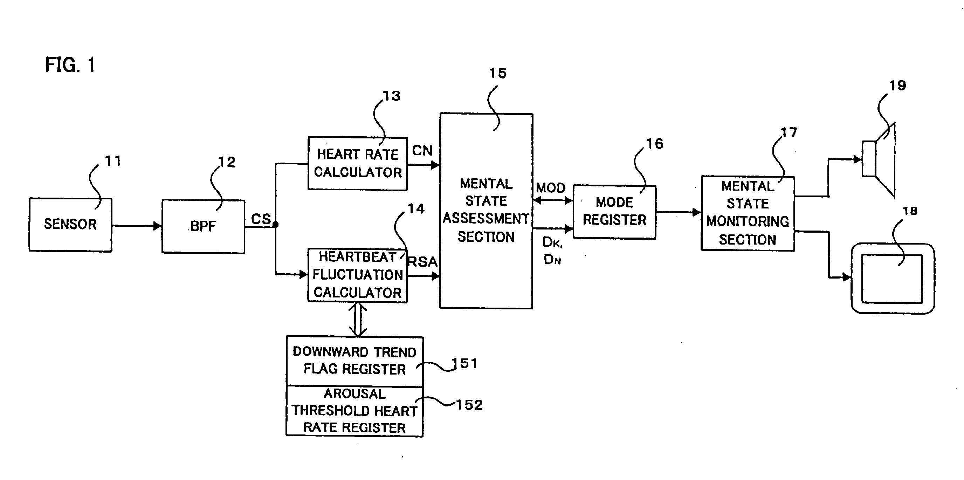

FIG. 1 is a diagram showing a configuration of a mental state assessment apparatus according to the present invention.

In FIG. 1, a sensor 11 supplies a BPF (Band Pass Filter) 12 with an electrical signal which corresponds to a potential difference between two appropriate points on a skin surface of a subject, or an electrical signal which corresponds to skin vibration which occurs on the skin surface of the subj ect or corresponds to heartbeats resulting from blood flow of the subject. The BPF 12 extracts only signal component which is given off by the action current generated by excitation of heart muscles and supplies them to a heart rate calculator 13 and heartbeat fluctuation calculator 14 as a myocardial pulse signal CS (electrocardiogram signal). The heart rate calculator 13 calculates heart rate based on the myocardial pulse signal CS and supplies heart rate data CN on the heart rate to a mental state assessment section 15 sequentially at regular intervals. The heartbeat fluctuation calculator 14 measures a 0.15 to 0.4Hz component of a time variation component in time intervals of the myocardial pulse signal CS as an HF (high frequency) value of the heartbeats and supplies heartbeat fluctuation data RSA which represents the heartbeats' HF value to the mental state assessment section 15 sequentially at regular intervals.

The mental state assessment section 15 assesses what mental state the subj ect is in--a slackened state (hereinafter referred to as a "sleepy state") in which the subj ect is sleepy, tense state, or normal state--by performing a mental state assessment process (described later) on the heart rate data CN and heartbeat fluctuation data RSA. Then, the mental state assessment section 15 supplies a mode signal MOD which represents the assessed mental state to a mode register 16. For example, it supplies the mode register 16 with a value of "0" if the subject is determined to be in a normal state, "1" if the subject is determined to be in a tense state, or "2" if the subject is determined to be in a sleepy state. Also, if the subject is in a tense state, the mental state assessment section 15 determines tension level $D_K$ and supplies it to the mode register 16. Furthermore, if the subject is in a sleepy state, the mental state assessment section 15 determines sleepiness level $D_N$ and supplies it to the mode register 16.

The mode register 16 separately stores the mode signal MOD, sleepiness level $D_N$, and tension level $D_K$ supplied from the mental state assessment section 15, by overwriting old data, and continues to supply them to a mental state monitoring section 17. Also, when a mode read command signal is supplied from the mental state assessment section 15, the mode register 16 supplies the mode signal MOD stored currently to the mental state assessment section 15.

The mental state monitoring section 17 generates an image signal to display images (including character images) which represent the mental state (sleepy state, tense state, normal state) indicated by the mode signal MOD as well as images which represent the sleepiness level $D_N$ or tension level $D_K$ and supplies it to a display 18. The display 18 presents the images based on the image signal on a screen. Also, the mental state monitoring section 17 supplies a speaker 19 with an audio signal at regular intervals to reproduce the mode signal MOD as well as the sleepiness level $D_N$ or tension level $D_K$ as sounds. The speaker 19 produces the sounds based on the audio signal. Alternatively, the mental statemonitoring section 17 may supply an image signal to the display 18 to display an image prompting a user to wake up and may supply an audio signal to the speaker 19 to produce sounds repeatedly prompting the user to wake up only when a mode signal MOD which represents a sleepy state is supplied.

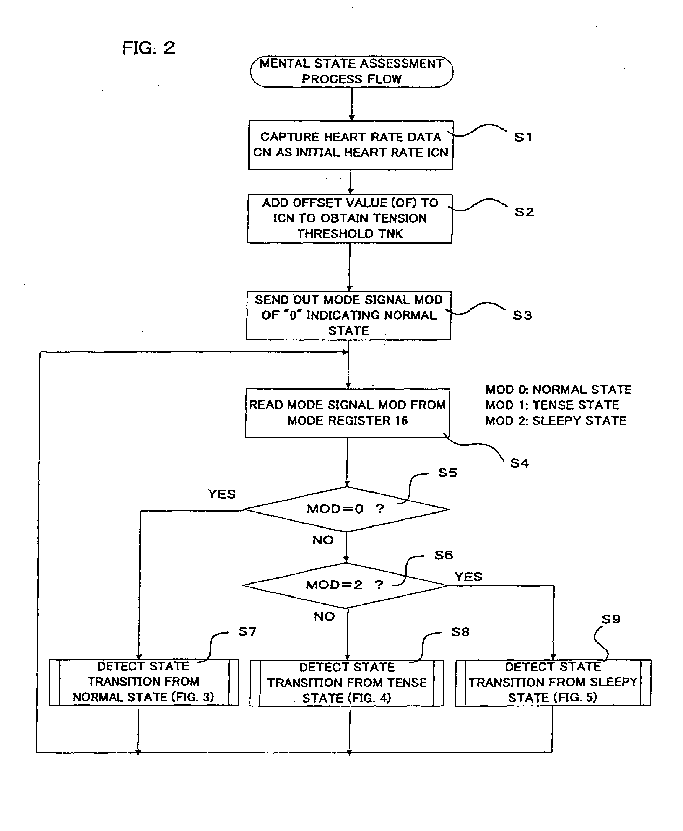

FIG. 2 is a diagram showing a flow of the mental state assessment process performed by the mental state assessment section 15 when measurement is started.

In FIG. 2, first the mental state assessment section 15 captures the heart rate data CN supplied from the heart rate calculator 13 and stores the heart rate indicated by the heart rate data CN as an initial heart rate ICN in a built-in memory (not shown) (Step S1). Next, the mental state assessment section 15 stores a result of adding a predetermined offset value OF to the initial heart rate ICN in the built-in memory as a tension threshold $T_{NK}$ for use to distinguish between normal state or sleepy state and tense state (Step S2). Then, the mental state assessment section 15 supplies a mode signal MOD of "0" which represents a normal state to the mode register 16 (Step S3). In response to execution of Step S3, the mental state monitoring section 17 supplies the display 18 with an image signal to display images (including character images) which represent a normal state. Next, the mental state assessment section 15 sends out a mode read command signal to the mode register 16, and thereby captures the mode signal MOD currently stored in the mode register 16 (Step S4).

Next, the mental state assessment section 15 determines whether the mode signal MOD captured in Step S4 is "0" indicating a normal state (Step S5). If it is found that the mode signal MOD is not "0," the mental state assessment section 15 determines whether it is "2" indicating a sleepy state (Step S6).

If it is found in Step S5 that the mode signal MOD is "0" indicating a normal state, the mental state assessment section 15 runs a subroutine for detecting a state transition from normal state (Step S7).

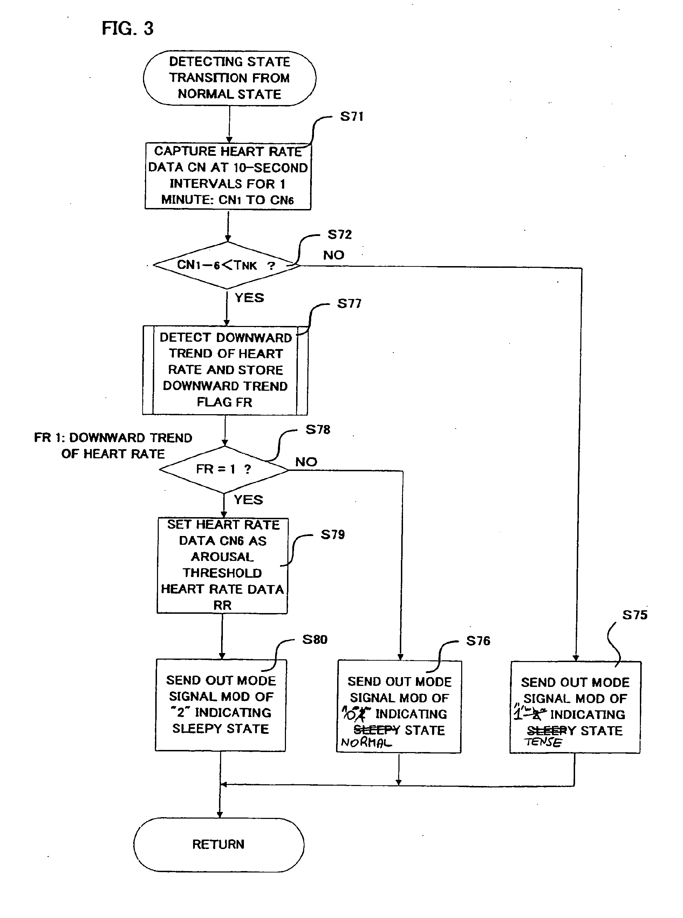

FIG. 3 shows a flow of the subroutine for detecting a state transition from normal state.

In FIG. 3, first the mental state assessment section 15 captures six samples of heart rate data CN sequentially at intervals of 10 seconds for 1 minute and stores them as heart rate data CN1 to CN6 in the built-in memory (Step S71). Specifically, it stores the heart rate data CN captured for 1 minute in the built-in memory sequentially by designating the heart rate data CN captured first as CN1, the heart rate data CN captured second as CN2, heart rate data CN captured third as CN3, heart rate data CN captured fourth as CN4, heart rate data CN captured fifth as CN5, and heart rate data CN captured sixth as CN6. Then, the mental state assessment section 15 determines whether all the heart rate data CN1 to CN6 are lower than the tension threshold $T_{NK}$ (Step S72). If it is found in Step S72 that all the heart rate data CN1 to CN6 are not lower than the tension threshold $T_{NK}$, the mental state assessment section 15 supplies the mode register 16 with a mode signal MOD of "1" indicating a tense state (Step S75). In response to execution of Step S75, the mental state monitoring section 17 supplies an image signal to the display 18 to display images (including character images) which represent a tense state. Thus, when Step S75 is executed, the display 18 presents the images which represent a tense state.

On the other hand, if it is found in Step S72 that all the heart rate data CN1 to CN6 are lower than the tension threshold $T_{NK}$, the mental state assessment section 15 detects whether the heart rate is on a downward trend, based on the heart rate data CN1 to CN6. The mental state assessment section 15 stores a downward trend flag FR of logic 1 in a downward trend flag register 151 if it detects that the heart rate is on a downward trend, but otherwise it stores a downward trend flag FR of logic 0 (Step S77).

When the heart rate downward trend detection process in Step S77 is finished, the mental state assessment section 15 determines whether the downward trend flag FR stored in the downward trend flag register 151 is logic 1 indicating a downward trend of the heart rate (Step S78). If it is found in Step S78 that the downward trend flag FR is not logic 1, i.e., if it is found in Step S78 that the heart rate is not on a downward trend, the mental state assessment section 15 goes to Step S76 as described above. That is, the mental state assessment section 15 supplies the mode register 16 with a mode signal MOD of "0" indicating a normal state.

On the other hand, if it is found in Step S78 that the downward trend flag FR is logic 1, i.e., if it is found in Step S78 that the heart rate is on a downward trend, the mental state assessment section 15 stores the heart rate data CN6 captured sixth in a arousal threshold heart rate register 152 as arousal threshold heart rate data RR (Step S79) . Then, the mental state assessment section 15 supplies the mode register 16 with a mode signal MOD of "2" indicating a sleepy state (Step S80). In response to execution of Step S80, the mental state monitoring section 17 supplies the display 18 with an image signal to display images (including character images) which represent a sleepy state and supplies an audio signal to the speaker 19 to produce sounds which represent the sleepy state. Alternatively, the mental state monitoring section 17 may supply an image signal to the display 18 to display an image prompting the user to wake up and may supply an audio signal to the speaker 19 to produce sounds prompting the user to wake up. Thus, the image presented on the display 18 upon execution of Step S80 represents the sleepy state (or prompts the user to wake up).

After the execution of Steps S75, S76, and S80, the mental state assessment section 15 returns to Step S4 in FIG. 2 and repeats the operations described above.

If it is found in Step S6 in FIG. 2 that the mode signal MOD indicates "1" which represents a tense state rather than "2" which represents a sleepy state, the mental state assessment section 15 runs a subroutine for detecting a state transition from tense state (Step S8).

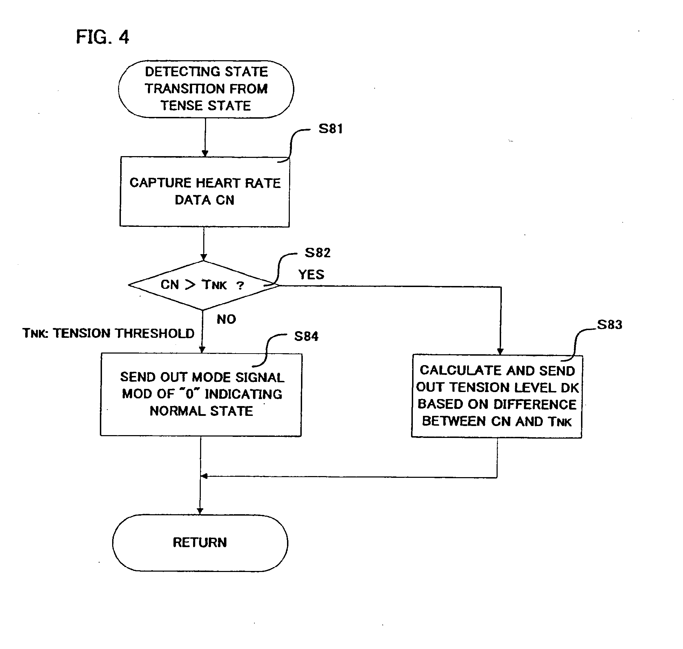

FIG. 4 shows a flow of the subroutine for detecting a state transition from tense state.

In FIG. 4, the mental state assessment section 15 captures one sample of heart rate data CN (Step S81) and determines whether its value is larger than the tension threshold $T_{NK}$ (Step S82). If it is found in Step S82 that the value is larger than the tension threshold $T_{NK}$, the mental state assessment section 15 adds a predetermined sensitivity coefficient to difference between the captured heart rate data CN and the tension threshold $T_{NK}$ and supplies the result of addition to the mode register 16 as the tension level $D_K$ (Step S83). In response to execution of Step S83, the mental state monitoring section 17 supplies the display 18 with an image signal to display images (including character images) which represent a tense state and an image which represents the tension level. Thus, as a result of Step S83, the display 18 presents the images which represent the tense state and tension level.

On the other hand, if it is found in Step S82 that the value of the heart rate data CN is not larger than the tension threshold $T_{NK}$, the mental state assessment section 15 supplies the mode register 16 with a mode signal MOD of "0" indicating a normal state (Step S84). In response to execution of Step S84, the mental state monitoring section 17 supplies the display 18 with an image signal to display images (including character images) which represent a normal state. Thus, as a result of Step S84, the display 18 presents the images which represent the normal state.

After the execution of Step S83 or S84, the mental state assessment section 15 returns to Step S4 in FIG. 2 and repeats the operations described above.

In Step S6 in FIG. 2, if the mode signal MOD indicates "2" which represents a sleepy state, the mental state assessment section 15 runs a subroutine for detecting a state transition from sleepy state (Step S9).

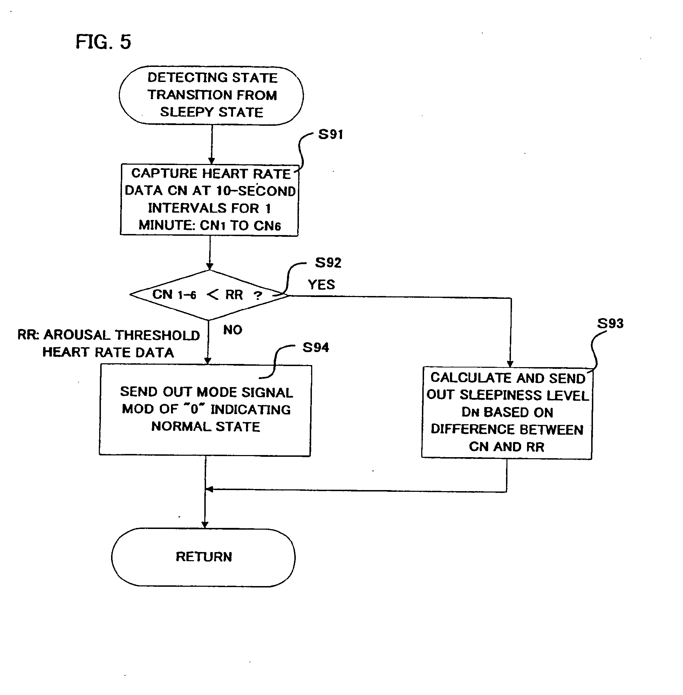

FIG. 5 shows a flow of the subroutine for detecting a state transition from sleepy state.

In FIG. 5, first the mental state assessment section 15 captures six samples of heart rate data CN sequentially at intervals of 10 seconds for 1 minute and stores them as heart rate data CN1 to CN6 in the built-in memory (Step S91) . Then, the mental state assessment section 15 determines whether all the heart rate data CN1 to CN6 are lower than the arousal threshold heart rate data RR stored in the arousal threshold heart rate register 152 (Step S92). If it is found in Step S92 that all the heart rate data CN1 to CN6 are lower than the arousal threshold heart rate data RR, the mental state assessment section 15 multiplies difference between the captured heart rate data CN and the arousal threshold heart rate data RR by a predetermined sensitivity coefficient and supplies the result of multiplication to the mode register 16 as the sleepiness level $D_N$ (Step S93). In response to execution of Step S93, the mental state monitoring section 17 supplies the display 18 with an image signal to display images (including character images) which represent a sleepy state and an image which represents the sleepiness level. Thus, as a result of Step S93, the display 18 presents the images which represent the sleepy state and sleepiness level. Furthermore the mental state monitoring section 17 supplies the speaker 19 with an audio signal to produce sounds which represent the sleepy state. Alternatively, the mental state monitoring section 17 may supply an image signal to the display 18 to display an image prompting the user to wake up and may supply an audio signal to the speaker 19 to produce sounds prompting the user to wake up.

On the other hand, if it is found in Step S92 that all the heart rate data CN1 to CN6 are not lower than the arousal threshold heart rate data RR, the mental state assessment section 15 supplies the mode register 16 with a mode signal MOD of "0" indicating a normal state (Step S94) . In response to execution of Step S94 , the mental state monitoring section 17 supplies an image signal to the display 18 to display images (including character images) which represent a normal state. Thus, when Step S94 is executed, the display 18 presents the images which represent a normal state.

After the execution of Step S93 or S94 , the mental state assessment section 15 returns to Step S4 in FIG. 2 and repeats the operations described above.

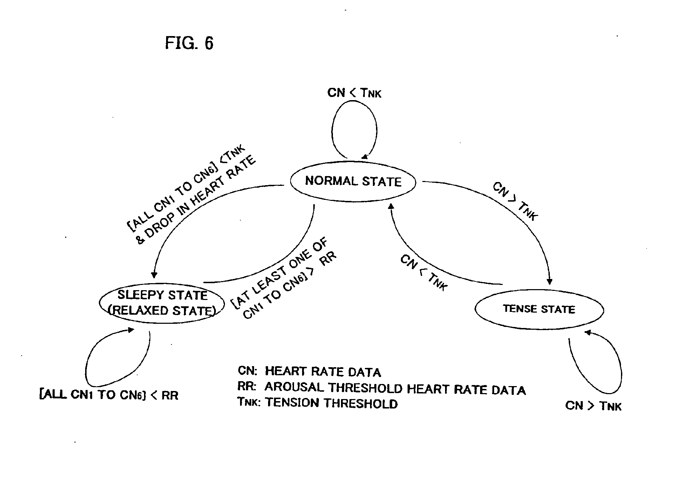

Mental state assessment operation shown in FIGS. 2 to 5 will be described below reference to FIG. 6.

If the subject was in a tense state to the last moment, the mental state assessment section 15 determines whether the mental state of the subject remains tense or has changed to normal state, based on the process of detecting a state transition from tense state in Step S8 (FIG. 4). If it is found in Step S82 that the heart rate data CN which represents the heart rate of the subject is higher than the tension threshold $T_{NK}$, the mental state assessment section 15 determines that the subject remains in a tense state as shown in FIG. 6. Incidentally, the tension threshold $T_{NK}$ is a value obtained by adding a predetermined offset value OF to the subject's initial heart rate measured at the beginning of measurement. On the other hand, if the heart rate data CN is lower than the tension threshold $T_{NK}$, the mental state assessment section 15 goes to Step S84, where it judges that the subject's mental state has changed from tense to normal as shown in FIG. 6.

If the subject was in a sleepy state (slackened state) to the last moment, the mental state assessment section 15 determines whether the mental state of the subject remains sleepy or has changed to normal state, based on the process of detecting a state transition from sleepy state in Step S9 (FIG. 5). If it is found in Step S92 that all the heart rate data CN1 to CN6 which represent the heart rate for one minute are lower than a arousal threshold heart rate represented by the arousal threshold heart rate data RR, the mental state assessment section 15 judges that the subject remains in a sleepy state (slackened state) as shown in FIG. 6. On the other hand, if it is found in Step S92 that all the heart rate data CN1 to CN6 are not lower than the arousal threshold heart rate data RR, the mental state assessment section 15 goes to Step S94, where it judges that the subj ect' s mental state has changed from sleepy (slackened) to normal as shown in FIG. 6. Incidentally, the arousal threshold heart rate data RR is set in the process of detecting a state transition from normal state in Step S79 and is the last heart rate data CN6 among the heart rate data series (CN1 to CN6) which is on a downward trend.

If the subject was in a normal state to the last moment, the mental state assessment section 15 determines whether the mental state of the subject remains normal or has changed to sleepy (slackened) or tense state, based on the process of detecting a state transition from normal state in Step S7 (FIG. 3) . If it is found in Step S72 that the heart rate data CN which represent the heart rate of the subject remain equal to or higher than the tension threshold $T_{NK}$ for one minute, the mental state assessment section 15 goes to Step S75, where it judges that the subject's mental state has changed from normal to tense state as shown in FIG. 6. On the other hand, if it is found that the heart rate data CN remain lower than the tension threshold $T_{NK}$ for one minute and it is found in Step S78 that the heart rate is not on a downward trend, the mental state assessment section 15 goes to Step S76, where it judges that the subj ect' s mental state remains normal as shown in FIG. 6. Besides, if it is found that the heart rate data CN remain lower than the tension threshold $T_{NK}$ for one minute and it is found in Step S78 that the heart rate is on a downward trend, the mental state assessment section 15 goes to Step S80, where it judges that the subject's mental state has changed from normal to sleepy (slackened) as shown in FIG. 6.

The present invention detects the downward trend of the subject's heart rate in view of the fact that when the mental state of a person changes from normal to slackened state in which the person feels sleepy, his/her heart rate either falls continuously or falls with up-and-down fluctuations.

Thus, the present invention can judge correctly that the subject has changed from normal state to sleepy state (slackened state) even if the average heart rate of the subj ect in normal state varies depending on external environment, time of day, etc.

Incidentally, since the heart rate also falls during transition from tense state to normal state, if the mental state is assessed based solely on the downward trend of the heart rate, there is a possibility to judge wrongly that the subject has entered a sleepy state when actually the subject is not sleepy. Thus, sleepy state is determined based on the downward trend of the heart rate only when it is found in Step S72 that the heart rate remains lower than the tension threshold $T_{NK}$ for one minute, i. e. , only when it is determined that the subject is in a state other than a tense state (i.e. , normal state or sleepy state). This makes it possible to avoid making a mistake of interpreting a transition from tense state to normal state as a transition from normal state to sleepy state.

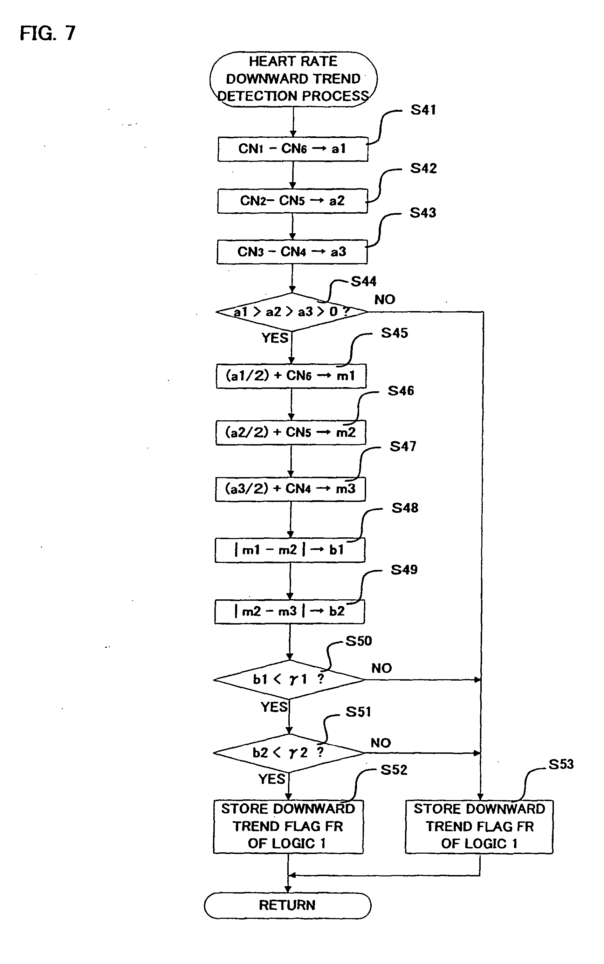

Here, a heart rate downward trend detection subroutine shown in FIG. 7 may be used to detect the downward trend of the heart rate correctly in Step S77 in FIG. 3 taking into consideration up-and-down fluctuations and noise.

In FIG. 7, first the mental state assessment section 15 finds difference in heart rate between the heart rate data CN1 captured first and heart rate data CN6 captured sixth out of the heart rate data CN1 to CN6 captured in Step S71 in FIG. 3 and stores it as a fall a1 in the built-in memory (Step S41). Next, themental state assessment section 15 finds difference in heart rate between the heart rate data CN2 captured second and heart rate data CN5 captured fifth and stores it as a fall a2 in the built-in memory (Step S42). Then, the mental state assessment section 15 finds difference in heart rate between the heart rate data CN3 captured third and heart rate data CN4 captured fourth and stores it as a fall a3 in the built-in memory (Step S43).

Next, themental state assessment section 15 determines whether the falls a1 to a3 satisfy a magnitude relationship (Step S44):

$$a1 > a2 > a3 > 0.$$

If it is found in Step S44 that the falls a1 to a3 satisfy the magnitude relationship, the mental state assessment section 15 finds a central value between the heart rate represented by the heart rate data CN1 and heart rate represented by the heart rate data CN6 and stores it as a central value m1 in the built-in memory (Step S45). Next, the mental state assessment section 15 finds a central value between the heart rate represented by the heart rate data CN2 and heart rate represented by the heart rate data CN5 and stores it as a central value m2 in the built-in memory (Step S46). Then, themental state assessment section 15 finds a central value between the heart rate represented by the heart rate data CN3 and heart rate represented by the heart rate data CN4 and stores it as a central value m3 in the built-in memory (Step S47). Next, the mental state assessment section 15 finds an absolute value of difference between the central value m2 and central value m1 and stores it as a variation b1 in the built-in memory (Step S48). Then, the mental state assessment section 15 finds an absolute value of difference between the central value m3 and central value m2 and stores it as a variation b2 in the built-in memory (Step S49). Next, the mental state assessment section 15 determines whether the variation b1 is smaller than a predetermined first reference value $\gamma1$ (Step S50). If it is found in Step S50 that the variation b1 is smaller than the first reference value $\gamma1$, the mental state assessment section 15 determines whether the variation b2 is smaller than a predetermined second reference value $\gamma2$ ($\gamma1 > \gamma2$) (Step S51). If it is found in Step S51 that the variation b2 is smaller than the second reference value $\gamma2$, the mental state assessment section 15 stores the downward trend flag FR of logic 1 in the downward trend flag register 151, indicating that the heart rate is on a downward trend (Step S52).

On the other hand, if it is found in Step S51 that the variation b2 is not smaller than the predetermined second reference value $\gamma2$ or if it is found in Step S50 that the variation b1 is not smaller than the predetermined first reference value $\gamma1$, the mental state assessment section 15 stores the downward trend flag FR of logic 0 in the downward trend flag register 151, indicating that the heart rate is not on a downward trend (Step S53).

Incidentally, if it is found in Step S44 that the falls a1 to a3 do not satisfy the magnitude relationship a1 > a2 > a3 > 0, the mental state assessment section 15 also goes through Step S53 to store the downward trend flag FR of logic 0 in the downward trend flag register 151.

After Step S52 or S53, the mental state assessment section 15 exits the heart rate downward trend detection subroutine in FIG. 4 and goes to Step S78 in FIG. 3.

Description will be given below of a heart rate downward trend detection operation performed through the execution of the heart rate downward trend detection subroutine.

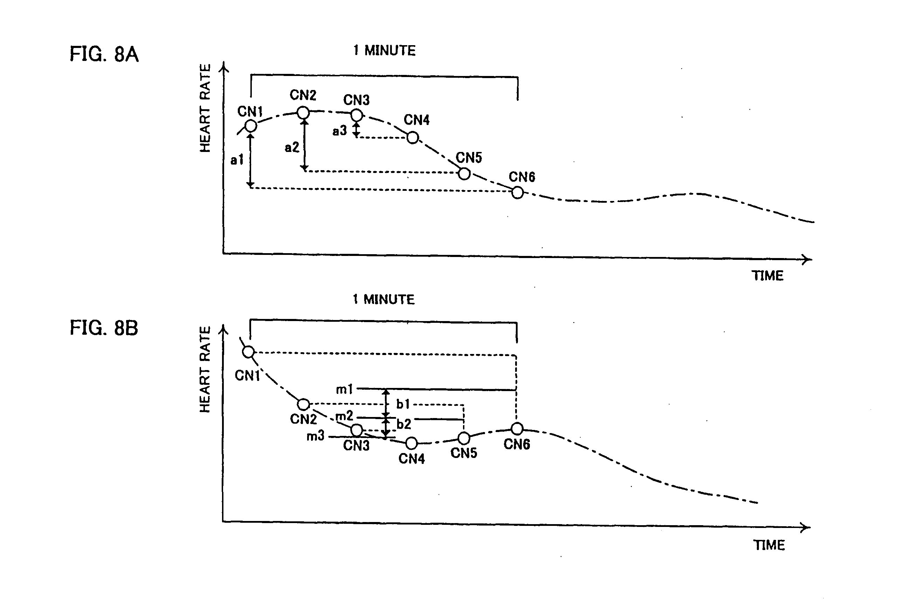

As the mental state of the subject changes from normal to sleepy (slackened), his/her heart rate falls gradually, fluctuating up and down as shown, for example, in FIG. 8A or 8B. Here, the fall a1 which corresponds to the difference between the heart rate data CN1 and CN6 out of the heart rate data CN1 to CN6 captured in one minute, the fall a2 which corresponds to the difference between the heart rate data CN2 and CN5 , and the fall a3 which corresponds to the difference between the heart rate data CN3 and CN4 satisfy the magnitude relationship:

$$a1 > a2 > a3 > 0.$$

That is, when the falls a1 to a3 do not satisfy the magnitude relationship, it can be said that the heart rate is not on a downward trend. Thus, if it is found in Step S44 that the falls a1 to a3 do not satisfy the magnitude relationship a1 > a2 > a3 > 0, the heart rate downward trend detection subroutine shown in FIG. 7 sets the downward trend flag FR to logic 0 in Step S53 to indicate that the heart rate is not on a downward trend.

However, even when the falls a1 to a3 satisfy the magnitude relationship, it cannot necessarily be said that the heart rate is on a downward trend if the heart rate fluctuates up and down greatly. For example, when the heart rate data CN1 to CN6 has a trend such as the one shown in FIG. 8B, if the heart rate fluctuates greatly within the one minute, the heart rate can show a significant upward trend between CN4 and CN6. Therefore, the heart rate may increase greatly after the heart rate data CN6. Thus, the heart rate downward trend detection subroutine shown in FIG. 7 determines in Step S50 whether the variation b1 represented by the difference between m1 andm2 is smaller than the first reference value $\gamma1$, where m1 is the central value between the heart rate data CN1 and CN6 while m2 is the central value between the heart rate data CN2 and CN5 , in the case of FIG. 8B, for example. Furthermore, the subroutine determines in Step S51 whether the variation b2 represented by the difference between m3 and m2 is smaller than the second reference value $\gamma2$, where m3 is the central value between the heart rate data CN3 and CN4 while m2 is the central value between the heart rate data CN2 and CN5 in FIG. 8B. If it is found in Steps S50 and S51 that the variation b1 is smaller than the first reference value $\gamma1$ and that the variation b2 is smaller than the second reference value $\gamma2$, the subroutine sets the downward trend flag FR to logic 1 to indicate that the heart rate is on a downward trend.

Thus, the heart rate downward trend detection subroutine shown in FIG. 7 judges that the heart rate is on a downward trend only if the fall a1 which corresponds to the difference between CN1 and CN6 out of the heart rate data CN1 to CN6 captured in one minute, the fall a2 which corresponds to the difference between CN2 and CN5, and the fall a3 which corresponds to the difference between CN3 and CN4 satisfy the magnitude relationship "a1 > a2 > a3 > 0" and the variation b1 represented by the difference between m1 and m2 as well as the variation b2 represented by the difference between m3 and m2 are smaller than the predetermined values γ1 and γ2, respectively, where m1 is the central value between CN1 and CN6, m2 is the central value between CN2 and CN5, and m3 is the central value between CN3 and CN4.

That is, when a value obtained by subtracting the heart rate at an end of the one minute from the heart rate acquired at a beginning of the one minute is larger than 0 and the variations of the heart rate within the one minute are smaller than the predetermined reference values, it is judged that the heart rate has a negative rate of change or is on a downward trend.

The heart rate downward trend detection process can detect heart rate correctly not only when it falls continuously, but also it falls with up-and-down fluctuations as shown in FIG. 8A or 8B. Also, since the heart rate downward trend detection process in FIG. 7 detects a downward trend based on a series of four or more heart rate data CN items, it can detect a downward trend of the heart rate correctly even if the value of one sample of heart rate data CN falls under influence of noise or the like.

Incidentally, when driving a vehicle, a driver may be both sleepy and tense simultaneously in terms of mental state. In such a case, even if the driver feels sleepy, his/her heart rate may not fall. However, when a sleepy state and tense state coexist, a transition of the driver's mental state to the sleepy state involves a rise in the heartbeats' HF value extracted from RSA (Respiratory Sinus Arrhythmia) which represents fluctuations in heartbeat intervals corresponding to respiratory variations. Thus, sleepy state may be assessed by detecting an upward trend of the heartbeats' HF value as described below instead of detecting a downward trend of heart rate.

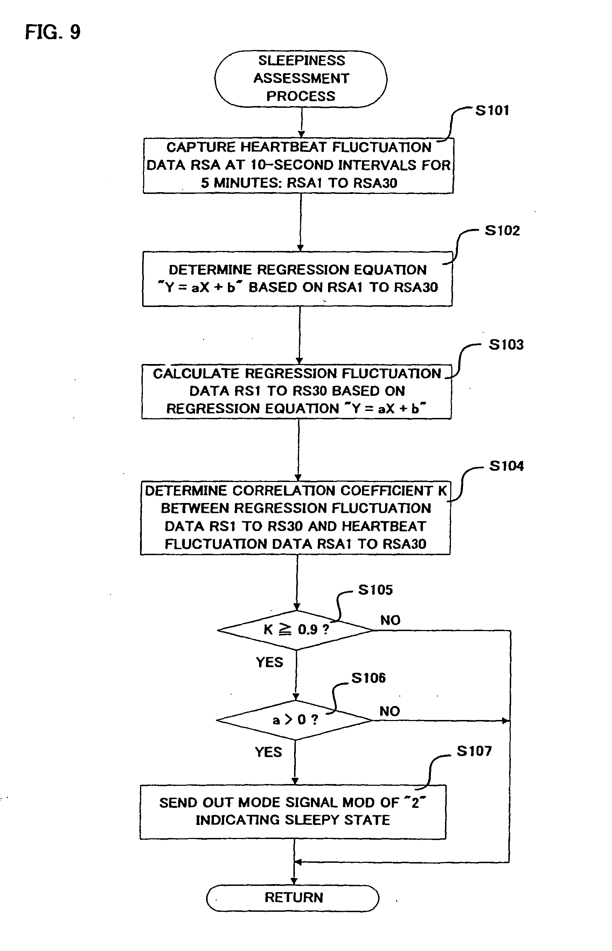

FIG. 9 shows a flow of a sleepiness assessment subroutine prepared in view of the above point. Incidentally, the sleepiness assessment subroutine shown in FIG. 9 is executed instead of Steps S77 to S80 after the execution of Steps S71 and S72 as shown in FIG. 3.

In FIG. 9, first the mental state assessment section 15 captures 30 samples of heartbeat fluctuation data RSA sequentially at intervals of 10 seconds for five minutes and stores them as heartbeat fluctuation data RSA1 to RSA30 in the built-in memory (Step S101). Next, by designating an X axis as a time axis of a data series consisting of the heartbeat fluctuation data RSA1 to RSA30, and a Y axis as a scale axis of the heartbeats' HF value, the mental state assessment section 15 determines a regression equation "Y = aX + b" which represents the data series (Step S102). Then, the mental state

assessment section 15 calculates regression fluctuation data RS1 to RS30 based on the regression equation "Y = aX + b" (Step S103). Then, the mental state assessment section 15 determines a correlation coefficient K between the regression fluctuation data RS1 to RS30 and heartbeat fluctuation data RSA1 to RSA30 (Step S104). Then, the mental state assessment section 15 determines whether the correlation coefficient K is equal to or higher than 0.9 (Step S105). If it is found in Step S105 that the correlation coefficient K is equal to or higher than 0.9, the mental state assessment section 15 determines whether a slope a of the regression equation is larger than 0, i.e., whether the slope a is positive (Step S106). If it is found in Step S106 that the slope a is positive, the mental state assessment section 15 determines that the heartbeats' HF value is on an upward trend and supplies the mode register 16 with a mode signal MOD of "2" indicating a sleepy state (Step S107).

After Step S107 is performed or if it is found in Step S105 that the correlation coefficient K is lower than 0.9 or if it is found in Step S106 that the slope a of the regression equation "Y = aX + b" is negative, the mental state assessment section 15 exits the sleepiness assessment subroutine and returns to Step S4.

The present invention has been described in detail by way of illustration and embodiments for purposes of clarity and understanding. However, it will be obvious that the present invention is not limited to the embodiments described herein, and that certain changes and modifications may be practiced within the scope of the invention, as limited only by the scope of the appended claims.

## Claims

1. A mental state assessment apparatus for assessing mental state of a subject, the assessment apparatus comprising.
   a sensor (11) for detecting from the subject a signal given off by an action current generated by excitation of cardiac muscles of the subject;
   a heart rate measuring device (13) for measuring heart rate based on the signal given off by the action current; and
   a sleepiness assessment device (15) for assessing that the subject is in a sleepy state if the heart rate continues to be lower than a predetermined value for a predetermined period and is on a downward trend;
   **characterised by**
   a setting device for setting the predetermined value by adding a predetermined offset value to the heart rate of the subject measured at a beginning of measurement,

2. The mental state assessment apparatus according to claim 1, wherein said sleepiness assessment device (15) includes heart rate downward trend detecting device for judging that the heart rate is on a downward trend if a result of subtracting the heart rate measured at an end of the predetermined period from the heart rate measured at a beginning of the predetermined period is larger than 0 and if magnitude of variation in the heart rate is smaller than a predetermined reference value.

3. The mental state assessment apparatus according to claim 2, wherein said heart rate downward trend detecting device comprises:

a first fall calculating device for calculating a first fall by subtracting the heart rate measured at a first time point in the predetermined period from the heart rate measured at a last time point in the predetermined period;
a second fall calculating device for calculating a second fall by subtracting the heart rate measured at a time point next to the first time point in the predetermined period from the heart rate measured at a time point just before the last time point in the predetermined period;
a first central value calculating device for calculating a first central value between the heart rate acquired at the first time point and the heart rate acquired at the last time point;
a second central value calculating device for calculating a second central value between the heart rate acquired at the time point next to the first time point and the heart rate measured at the time point just before the last time point;
a variation calculating device for calculating an absolute value of difference between the first central value and the second central value as variation; and
a device for judging that the heart rate is on a downward trend if the variation is smaller than a predetermined reference value and if the first fall and the second fall satisfy
first fall > second fall > 0.

4. The mental state assessment apparatus according to any of claims 1 to 3, further comprising a tension assessment device for judging that the subject is in a tense state if the heart rate is higher than the predetermined value.

5. The mental state assessment apparatus according to any of claims 1 to 4, wherein the predetermined period is one minute.

6. A mental state assessment method for assessing mental state of a subject, the method comprising:

a heart rate measuring process of measuring heart rate of the subject;

sleepiness assessment process of assessing that the subject is in a sleepy state if the heart rate continues to be lower than a predetermined value for a predetermined period and is on a downward trend;
**characterised by**
a setting process of setting the predetermined value by adding a predetermined offset value to the heart rate of the subject measured at a beginning of measurement;

7. The mental state assessment method according to claim 6, wherein said sleepiness assessment process includes a heart rate downward trend detecting process of judging that the heart rate is on a downward trend if a result of subtracting the heart rate measured at an end of the predetermined period from the heart rate measured at a beginning of the predetermined period is larger than 0 and if magnitude of variation in the heart rate is smaller than a predetermined reference value.

8. The mental state assessment method according to claim 7, wherein said heart rate downward trend detecting process comprises:

a first fall calculating process of calculating a first fall by subtracting the heart rate measured at a first time point in the predetermined period from the heart rate measured at a last time point in the predetermined period;
a second fall calculating process of calculating a second fall by subtracting the heart rate measured at a time point next to the first time point in the predetermined period from the heart rate measured at a time point just before the last time point in the predetermined period;
a first central value calculating process of calculating a first central value between the heart rate acquired at the first time point and the heart rate acquired at the last time point;
a second central value calculating process of calculating a second central value between the heart rate acquired at the time point next to the first time point and the heart rate measured at the time point just before the last time point;
a variation calculating process of calculating an absolute value of difference between the first central value and the second central value as variation; and
a process of judging that the heart rate is on a downward trend if the variation is smaller than a predetermined reference value and if the first fall and the second fall satisfy
first fall > second fall > 0.

9. The mental state assessment method according to claim 8, further comprising a tension assessment process of judging that the subject is in a tense state if the heart rate is higher than the predetermined value.

10. The mental state assessment method according to any of claims 6 to 9, wherein the predetermined period is one minute.

**Patentansprüche**

1. Vorrichtung zur Bestimmung des geistigen Zustands eines Probanden, wobei die Bestimmungsvorrichtung folgendes umfasst:

einen Sensor (11) zur Erfassung eines Signals von dem Probanden, das durch einen durch die Erregung von Herzmuskeln des Probanden erzeugten Aktionsstrom abgegeben wird; eine Herzfrequenzmessvorrichtung (13) zur Messung der Herzfrequenz aufgrund des durch den Aktionsstrom abgegebenen Signals; und eine Schläfrigkeitsbestimmungsvorrichtung (15) zur Bestimmung, dass sich der Proband in einem schläfrigen Zustand befindet, wenn die Herzfrequenz während eines vorbestimmten Zeitraums anhaltend niedriger ist als ein vorbestimmter Wert und sie eine nach unten gehende Tendenz zeigt; **gekennzeichnet durch** eine Einstellvorrichtung zur Einstellung des vorbestimmten Wertes **durch** Addieren eines vorbestimmten Verschiebungswertes zur zu Beginn der Messung gemessenen Herzfrequenz des Probanden.

2. Vorrichtung zur Bestimmung des geistigen Zustands nach Anspruch 1, wobei die Schläfrigkeitsbestimmungsvorrichtung (15) eine Vorrichtung zur Erfassung der nach unten gehenden Tendenz der Herzfrequenz umfasst, zur Bewertung, dass die Herzfrequenz eine nach unten gehenden Tendenz zeigt, wenn ein Ergebnis des Subtrahierens der am Ende des vorbestimmten Zeitraums gemessenen Herzfrequenz von der zu Beginn des vorbestimmten Zeitraums gemessenen Herzfrequenz größer ist als 0, und wenn die Größenordnung der Veränderung in der Herzfrequenz kleiner ist als ein vorbestimmter Referenzwert.

3. Vorrichtung zur Bestimmung des geistigen Zustands nach Anspruch 2, wobei die Vorrichtung zur Erfassung der nach unten gehenden Tendenz der Herzfrequenz folgendes umfasst:

eine erste Abfallberechnungsvorrichtung zur Berechnung eines ersten Abfalls durch Subtrahieren der zu einem ersten Zeitpunkt in dem vorbestimmten Zeitraum gemessenen Herzfrequenz von der zu einem letzten Zeitpunkt in dem vorbestimmten Zeitraum gemessenen Herzfrequenz; eine zweite Abfallberechnungsvorrichtung zur Berechnung eines zweiten Abfalls durch Subtrahieren der zu einem dem ersten Zeitpunkt folgenden Zeitpunkt in dem vorbestimmten Zeitraum gemessenen Herzfrequenz von der zu einem dem letzten Zeitpunkt vorausgehenden Zeitpunkt in dem vorbestimmten Zeitraum gemessenen Herzfrequenz; eine erste Mittelwertberechnungsvorrichtung zur Berechnung eines ersten Mittelwertes zwischen der zu dem ersten Zeitpunkt erhaltenen Herzfrequenz und der zu dem letzten Zeitpunkt erhaltenen Herzfrequenz; eine zweite Mittelwertberechnungsvorrichtung zur Berechnung eines zweiten Mittelwertes zwischen der zu dem dem ersten Zeitpunkt folgenden Zeitpunkt erhaltenen Herzfrequenz und der zu dem dem letzten Zeitpunkt vorausgehenden Zeitpunkt gemessenen Herzfrequenz; eine Veränderungsberechnungsvorrichtung zur Berechnung eines Absolutwertes aus der Differenz zwischen dem ersten Mittelwert und dem zweiten Mittelwert als Veränderung; und eine Vorrichtung zur Bewertung, dass die Herzfrequenz eine nach unten gehenden Tendenz zeigt, wenn die Veränderung kleiner ist als ein vorbestimmter Referenzwert und wenn der erste Abfall und der zweite Abfall folgendes erfüllen: erster Abfall > zweiter Abfall > 0.

4. Vorrichtung zur Bestimmung des geistigen Zustands nach einem der Ansprüche 1 bis 3, weiterhin umfassend eine Spannungsbestimmungsvorrichtung zur Bewertung, dass sich der Proband in einem angespannten Zustand befindet, wenn die Herzfrequenz höher ist als der vorbestimmte Wert.

5. Vorrichtung zur Bestimmung des geistigen Zustands nach einem der Ansprüche 1 bis 4, wobei der vorbestimmte Zeitraum eine Minute beträgt.

6. Verfahren zur Bestimmung des geistigen Zustands eines Probanden, wobei das Verfahren folgendes umfasst:

ein Herzfrequenzmessverfahren zur Messung der Herzfrequenz des Probanden; ein Schläfrigkeitsbestimmungsverfahren zur Bestimmung, dass sich der Proband in einem schläfrigen Zustand befindet, wenn die Herzfrequenz während eines vorbestimmten Zeitraums anhaltend niedriger ist als ein vorbestimmter Wert und sie eine nach unten gehende Tendenz zeigt; **gekennzeichnet durch**

ein Einstellverfahren zur Einstellung des vorbestimmten Wertes **durch** Addieren eines vorbestimmten Verschiebungswertes zur zu Beginn der Messung gemessenen Herzfrequenz des Probanden.

**7.** Verfahren zur Bestimmung des geistigen Zustands nach Anspruch 6, wobei das Schläfrigkeitsbestimmungsverfahren ein Verfahren zur Erfassung der nach unten gehenden Tendenz der Herzfrequenz umfasst, zur Bewertung, dass die Herzfrequenz eine nach unten gehenden Tendenz zeigt, wenn ein Ergebnis des Subtrahierens der am Ende des vorbestimmten Zeitraums gemessenen Herzfrequenz von der zu Beginn des vorbestimmten Zeitraums gemessenen Herzfrequenz größer ist als 0, und wenn die Größenordnung der Veränderung in der Herzfrequenz kleiner ist als ein vorbestimmter Referenzwert.

**8.** Verfahren zur Bestimmung des geistigen Zustands nach Anspruch 7, wobei das Verfahren zur Erfassung der nach unten gehenden Tendenz der Herzfrequenz folgendes umfasst:

ein erstes Abfallberechnungsverfahren zur Berechnung eines ersten Abfalls durch Subtrahieren der zu einem ersten Zeitpunkt in dem vorbestimmten Zeitraum gemessenen Herzfrequenz von der zu einem letzten Zeitpunkt in dem vorbestimmten Zeitraum gemessenen Herzfrequenz;
ein zweites Abfallberechnungsverfahren zur Berechnung eines zweiten Abfalls durch Subtrahieren der zu einem dem ersten Zeitpunkt folgenden Zeitpunkt in dem vorbestimmten Zeitraum gemessenen Herzfrequenz von der zu einem dem letzten Zeitpunkt vorausgehenden Zeitpunkt in dem vorbestimmten Zeitraum gemessenen Herzfrequenz; ein erstes Mittelwertberechnungsverfahren zur Berechnung eines ersten Mittelwertes zwischen der zu dem ersten Zeitpunkt erhaltenen Herzfrequenz und der zu dem letzten Zeitpunkt erhaltenen Herzfrequenz; ein zweites Mittelwertberechnungsverfahren zur Berechnung eines zweiten Mittelwertes zwischen der zu dem dem ersten Zeitpunkt folgenden Zeitpunkt erhaltenen Herzfrequenz und der zu dem dem letzten Zeitpunkt vorausgehenden Zeitpunkt gemessenen Herzfrequenz; ein Veränderungsberechnungsverfahren zur Berechnung eines Absolutwertes aus der Differenz zwischen dem ersten Mittelwert und dem zweiten Mittelwert als Veränderung; und ein Verfahren zur Bewertung, dass die Herzfrequenz eine nach unten gehenden Tendenz zeigt, wenn die Veränderung kleiner ist als ein vorbestimmter Referenzwert und wenn der erste Abfall und der zweite Abfall folgendes erfüllen: erster Abfall > zweiter Abfall > 0.

**9.** Verfahren zur Bestimmung des geistigen Zustands nach Anspruch 8, weiterhin umfassend ein Spannungsbestimmungsverfahren zur Bewertung, dass sich der Proband in einem angespannten Zustand befindet, wenn die Herzfrequenz höher ist als der vorbestimmte Wert.

**10.** Verfahren zur Bestimmung des geistigen Zustands nach einem der Ansprüche 6 bis 9, wobei der vorbestimmte Zeitraum eine Minute beträgt.

## Revendications

**1.** Appareil d'évaluation de la condition mentale destiné à évaluer l'état mental d'un sujet, l'appareil d'évaluation comprenant :

- un capteur (11) destiné à détecter, à partir du sujet, un signal émis par un courant d'action généré par l'excitation des muscles cardiaques du sujet ;
- un dispositif de mesure du rythme cardiaque (13) destiné à mesurer le rythme cardiaque sur la base du signal émis par le courant d'action ; et
- un dispositif d'évaluation de la somnolence (15) destiné à évaluer que le sujet est dans un état de sommeil si le rythme cardiaque continue à être inférieur à une valeur prédéterminée sur une période prédéterminée et tend à la baisse ;

**caractérisé par** un dispositif de réglage destiné à régler la valeur prédéterminée en ajoutant une valeur de décalage prédéterminée au rythme cardiaque du sujet mesuré à un début de la prise de mesure.

**2.** Appareil d'évaluation de la condition mentale selon la revendication 1, dans lequel ledit dispositif d'évaluation de la somnolence (15) comprend le dispositif de détection de tendance à la baisse du rythme cardiaque destiné à estimer que le rythme cardiaque tend à la baisse si un résultat de la soustraction du rythme cardiaque mesuré à une fin de la période prédéterminée au rythme cardiaque mesuré à un début de la période prédéterminée est supérieur à 0 et si la magnitude de la variation du rythme cardiaque est inférieure à une valeur de référence prédéterminée.

**3.** Appareil d'évaluation de la condition mentale selon la revendication 2, dans lequel ledit dispositif de détection de la tendance à la baisse du rythme cardiaque comprend :

- un premier appareil de calcul de la première baisse destiné à calculer une première baisse en soustrayant le rythme cardiaque mesuré à un premier point d'échantillonnage dans la période prédéterminée au rythme cardiaque mesuré à un dernier point d'échantillonnage dans la période prédéterminée ;

- un second appareil de calcul de la seconde baisse destiné à calculer une seconde baisse en soustrayant le rythme cardiaque mesuré à un point d'échantillonnage après le premier point dans la période prédéterminée au rythme cardiaque mesuré à un point d'échantillonnage juste avant le dernier point d'échantillonnage dans la période prédéterminée ;

- un premier appareil de calcul de la première valeur centrale destiné à calculer une première valeur centrale entre le rythme cardiaque obtenu au premier point d'échantillonnage et le rythme cardiaque obtenu au dernier point d'échantillonnage ;

- un second appareil de calcul de la seconde valeur centrale destiné à calculer une seconde valeur centrale entre le rythme cardiaque obtenu au point d'échantillonnage après le premier point d'échantillonnage et le rythme cardiaque mesuré au point d'échantillonnage juste avant le dernier point d'échantillonnage ;

- un appareil de calcul de variation destiné à calculer une valeur absolue de différence entre la première valeur centrale et la seconde valeur centrale en tant que variation ; et

- un appareil destiné à estimer que le rythme cardiaque tend à la baisse si la variation est inférieure à une valeur de référence prédéterminée et si la première baisse et la seconde baisse répondent à

première baisse > seconde baisse > 0.

4. Appareil d'évaluation de la condition mentale selon l'une des revendications 1 à 3, comprenant en outre un dispositif d'évaluation de la tension destiné à estimer que le sujet est dans un état tendu si le rythme cardiaque est supérieur à la valeur prédéterminée.

5. Appareil d'évaluation de la condition mentale selon l'une des revendications 1 à 4, dans lequel la période prédéterminée est une minute.

6. Procédé d'évaluation de la condition mentale destiné à évaluer l'état mental d'un sujet, le procédé comprenant :

- un processus de mesure du rythme cardiaque conçu pour mesurer le rythme cardiaque du sujet ;

- un processus d'évaluation de la somnolence conçu pour évaluer que le sujet est dans un état de sommeil si le rythme cardiaque continue à être inférieur à une valeur prédéterminée sur une période prédéterminée et tend à la baisse ;

**caractérisé par** un processus de réglage destiné à régler la valeur prédéterminée en ajoutant une valeur de décalage prédéterminée au rythme cardiaque du sujet mesuré à un début de la prise de mesure.

7. Procédé d'évaluation de la condition mentale selon la revendication 6, dans lequel ledit processus d'évaluation de la somnolence comprend un processus de détection de la tendance à la baisse du rythme cardiaque destiné à estimer que le rythme cardiaque tend à la baisse si un résultat de la soustraction du rythme cardiaque mesuré à une fin de la période prédéterminée au rythme cardiaque mesuré à un début de la période prédéterminée est supérieur à 0 et si la magnitude de la variation du rythme cardiaque est inférieure à une valeur de référence prédéterminée.

8. Procédé d'évaluation de la condition mentale selon la revendication 7, dans lequel ledit processus de détection de la tendance à la baisse du rythme cardiaque comprend :

- un premier processus de calcul de la première baisse conçu pour calculer une première baisse en soustrayant le rythme cardiaque mesuré à un premier point d'échantillonnage dans la période prédéterminée au rythme cardiaque mesuré à un dernier point d'échantillonnage dans la période prédéterminée ;

- un second processus de calcul de la seconde baisse conçu pour calculer une seconde baisse en soustrayant le rythme cardiaque mesuré à un point d'échantillonnage après le premier point d'échantillonnage dans la période prédéterminée au rythme cardiaque mesuré à un point d'échantillonnage juste avant le dernier point d'échantillonnage dans la période prédéterminée ;

- un premier processus de calcul de la première valeur centrale conçu pour calculer une première valeur centrale entre le rythme cardiaque obtenu au premier point d'échantillonnage et le rythme cardiaque obtenu au dernier point d'échantillonnage ;

- un second processus de calcul de la seconde valeur centrale conçu pour calculer une seconde valeur centrale entre le rythme cardiaque obtenu au point d'échantillonnage après le premier point d'échantillonnage et le rythme cardiaque mesuré au point d'échantillonnage juste avant le dernier point d'échantillonnage ;

- un processus de calcul de variation conçu pour calculer une valeur absolue de différence entre la première valeur centrale et la seconde valeur centrale en tant que variation ; et

- un processus conçu pour estimer que le rythme cardiaque tend à la baisse si la variation est inférieure à une valeur de référence prédéterminée et si la première baisse et la seconde baisse répondent à première baisse > seconde baisse > 0.

9. Procédé d'évaluation de la condition mentale selon la revendication 8, comprenant en outre un processus d'évaluation de la tension conçu pour estimer que le sujet est dans un état tendu si le rythme cardiaque est supérieur à la valeur prédéterminée.

10. Procédé d'évaluation de la condition mentale selon l'une des revendications 6 à 9, dans lequel la période prédéterminée est une minute.

FIG. 1

FIG. 2

```
        ( MENTAL STATE ASSESSMENT )
        (    PROCESS FLOW        )
                  |
                  v
        +----------------------+
        | CAPTURE HEART RATE DATA |  ~~~ S1
        | CN AS INITIAL HEART RATE ICN |
        +----------------------+
                  |
                  v
        +----------------------+
        | ADD OFFSET VALUE (OF) TO |  ~~~ S2
        | ICN TO OBTAIN TENSION   |
        | THRESHOLD TNK           |
        +----------------------+
                  |
                  v
        +----------------------+
        | SEND OUT MODE SIGNAL MOD |  ~~~ S3
        | OF "0" INDICATING NORMAL |
        | STATE                   |
        +----------------------+
                  |
                  v
        +----------------------+        MOD 0: NORMAL STATE
        | READ MODE SIGNAL MOD FROM |   MOD 1: TENSE STATE
        | MODE REGISTER 16        |     MOD 2: SLEEPY STATE
        +----------------------+
                  |                 ~~~ S4
                  v
          S5 ___
             <  MOD=0 ? >
  YES  <----       |
                  NO
                   |
                   v
          S6 ___
             <  MOD=2 ? >  YES ---->
                   |
                  NO
                   |
```

| DETECT STATE TRANSITION FROM NORMAL STATE (FIG. 3) — S7 | DETECT STATE TRANSITION FROM TENSE STATE (FIG. 4) — S8 | DETECT STATE TRANSITION FROM SLEEPY STATE (FIG. 5) — S9 |

## FIG. 3

DETECTING STATE TRANSITION FROM NORMAL STATE

S71
CAPTURE HEART RATE DATA CN AT 10-SECOND INTERVALS FOR 1 MINUTE: CN1 TO CN6

S72
$CN1-6 < T_{NK}$ ?
NO
YES

S77
DETECT DOWNWARD TREND OF HEART RATE AND STORE DOWNWARD TREND FLAG FR

FR 1: DOWNWARD TREND OF HEART RATE

S78
FR = 1 ?
NO
YES

S79
SET HEART RATE DATA CN6 AS AROUSAL THRESHOLD HEART RATE DATA RR

S80
SEND OUT MODE SIGNAL MOD OF "2" INDICATING SLEEPY STATE

S76
SEND OUT MODE SIGNAL MOD OF "0" INDICATING NORMAL STATE

S75
SEND OUT MODE SIGNAL MOD OF "1" INDICATING TENSE STATE

RETURN

FIG. 4

```
      ┌─────────────────────┐
      │  DETECTING STATE    │
      │  TRANSITION FROM    │
      │  TENSE STATE        │
      └─────────────────────┘
                │
                ▼                    ─ S81
      ┌─────────────────────┐
      │  CAPTURE HEART RATE │
      │  DATA CN            │
      └─────────────────────┘
                │
                ▼            ─ S82
              ╱─────────╲         YES
             ⟨ CN > TNK ? ⟩──────────────┐
              ╲─────────╱                │
                │ NO                     │
TNK: TENSION THRESHOLD │    ─ S84        │              ─ S83
                ▼                        ▼
      ┌─────────────────────┐  ┌─────────────────────┐
      │ SEND OUT MODE SIGNAL│  │ CALCULATE AND SEND  │
      │ MOD OF "0" INDICATING│ │ OUT TENSION LEVEL DK│
      │ NORMAL STATE        │  │ BASED ON DIFFERENCE │
      └─────────────────────┘  │ BETWEEN CN AND TNK  │
                │              └─────────────────────┘
                │◄──────────────────────┘
                ▼
      ┌─────────────────────┐
      │      RETURN         │
      └─────────────────────┘
```

FIG. 5

```
    ┌─────────────────────────┐
   (  DETECTING STATE          )
   (  TRANSITION FROM          )
   (  SLEEPY STATE             )
    └────────────┬────────────┘
                 │                     ⌐ S91
                 ▼
   ┌─────────────────────────┐
   │ CAPTURE HEART RATE       │
   │ DATA CN AT 10-SECOND     │
   │ INTERVALS FOR 1          │
   │ MINUTE: CN1 TO CN6       │
   └────────────┬────────────┘
                │                      ⌐ S92
                ▼                        YES
          ◇─────────────◇──────────────────────┐
           < CN 1-6 < RR ? >                    │
          ◇─────────────◇                       │
                │                                │
RR: AROUSAL THRESHOLD   │ NO                     │            ⌐ S93
HEART RATE DATA         │         ⌐ S94         │
                        ▼                        ▼
          ┌─────────────────────┐   ┌─────────────────────┐
          │ SEND OUT MODE SIGNAL │   │ CALCULATE AND SEND   │
          │ MOD OF "0" INDICATING│   │ OUT SLEEPINESS LEVEL │
          │ NORMAL STATE         │   │ DN BASED ON          │
          │                      │   │ DIFFERENCE BETWEEN   │
          │                      │   │ CN AND RR            │
          └──────────┬──────────┘   └──────────┬──────────┘
                     │                          │
                     ◄──────────────────────────┘
                     │
                     ▼
          ┌─────────────────────┐
         (     RETURN            )
          └─────────────────────┘
```

FIG. 6

CN: HEART RATE DATA
RR: AROUSAL THRESHOLD HEART RATE DATA
$T_{NK}$: TENSION THRESHOLD

## FIG. 7

```
        HEART RATE
      DOWNWARD TREND
    DETECTION PROCESS
              │
              │                    S41
              ▼
      ┌─────────────────┐
      │  CN1 − CN6 → a1  │
      └─────────────────┘
              │                    S42
              ▼
      ┌─────────────────┐
      │  CN2− CN5 → a2   │
      └─────────────────┘
              │                    S43
              ▼
      ┌─────────────────┐
      │  CN3 − CN4 → a3  │
      └─────────────────┘
              │          S44
              ▼               NO
      ◇ a1 > a2 > a3 > 0 ? ◇──────────────┐
              │                           │
             YES            S45           │
              ▼                           │
      ┌─────────────────┐                 │
      │ (a1/2) + CN6 → m1│                 │
      └─────────────────┘                 │
              │               S46         │
              ▼                           │
      ┌─────────────────┐                 │
      │ (a2/2) + CN5 → m2│                 │
      └─────────────────┘                 │
              │                  S47       │
              ▼                           │
      ┌─────────────────┐                 │
      │ (a3/2) + CN4 → m3│                 │
      └─────────────────┘                 │
              │               S48         │
              ▼                           │
      ┌─────────────────┐                 │
      │  | m1 − m2 | → b1│                 │
      └─────────────────┘                 │
              │               S49         │
              ▼                           │
      ┌─────────────────┐                 │
      │  | m2 − m3 | → b2│                 │
      └─────────────────┘                 │
              │        S50                │
              ▼              NO           │
      ◇   b1 < γ1 ?   ◇──────────────────►│
              │                           │
             YES          S51             │
              ▼              NO           │
      ◇   b2 < γ2 ?   ◇──────────────────►│
              │                           │
             YES      S52              S53│
              ▼                           ▼
   ┌──────────────────┐      ┌──────────────────┐
   │  STORE DOWNWARD  │      │  STORE DOWNWARD  │
   │  TREND FLAG FR   │      │  TREND FLAG FR   │
   │   OF LOGIC 1     │      │   OF LOGIC 1     │
   └──────────────────┘      └──────────────────┘
              │                      │
              ▼◄─────────────────────┘
        ┌───────────┐
        │  RETURN   │
        └───────────┘
```

FIG. 8A

1 MINUTE

HEART RATE

CN1
CN2
CN3
CN4
CN5
CN6

a1
a2
a3

TIME

FIG. 8B

1 MINUTE

HEART RATE

CN1
CN2
m1
m2
b1
m3
CN3
CN4
CN5
CN6
b2

TIME

EP 1 479 342 B1

## FIG. 9

```
        ┌─────────────────────┐
        │    SLEEPINESS        │
        │    ASSESSMENT        │
        │    PROCESS           │
        └─────────────────────┘
                  │
                  ▼
   ┌──────────────────────────────────┐  ⎞─ S101
   │  CAPTURE HEARTBEAT FLUCTUATION    │
   │ DATA RSA AT 10-SECOND INTERVALS FOR│
   │     5 MINUTES: RSA1 TO RSA30      │
   └──────────────────────────────────┘
                  │
                  ▼
   ┌──────────────────────────────────┐  ⎞─ S102
   │    DETERMINE REGRESSION EQUATION  │
   │  "Y = aX + b" BASED ON RSA1 TO RSA30│
   └──────────────────────────────────┘
                  │
                  ▼
   ┌──────────────────────────────────┐  ⎞─ S103
   │ CALCULATE REGRESSION FLUCTUATION  │
   │     DATA RS1 TO RS30 BASED ON     │
   │  REGRESSION EQUATION "Y = aX + b" │
   └──────────────────────────────────┘
                  │
                  ▼
   ┌──────────────────────────────────┐  ⎞─ S104
   │ DETERMINE CORRELATION COEFFICIENT K│
   │  BETWEEN REGRESSION FLUCTUATION   │
   │   DATA RS1 TO RS30 AND HEARTBEAT  │
   │   FLUCTUATION DATA RSA1 TO RSA30  │
   └──────────────────────────────────┘
                  │
                  ▼
              ◇ S105
         ┌─────────────┐    NO
         │  K ≧ 0.9 ?  │────────────┐
         └─────────────┘            │
              │ YES                 │
              ▼                     │
              ◇ S106               │
         ┌─────────────┐    NO      │
         │   a > 0 ?   │───────────▶│
         └─────────────┘            │
              │ YES                 │
              ▼         S107        │
   ┌──────────────────────────────┐ │
   │ SEND OUT MODE SIGNAL MOD OF "2"│ │
   │    INDICATING SLEEPY STATE    │ │
   └──────────────────────────────┘ │
              │                      │
              ▼◀─────────────────────┘
        ┌─────────────┐
        │   RETURN    │
        └─────────────┘
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H07059757 A

- DE 4400207 A